**Europäisches Patentamt**

**European Patent Office**

**Office européen des brevets**

(19)

(11) Veröffentlichungsnummer: **0 044 498**
**B1**

(12) # EUROPÄISCHE PATENTSCHRIFT

(45) Veröffentlichungstag der Patentschrift:
**19.09.84**

(21) Anmeldenummer: **81105458.4**

(22) Anmeldetag: **13.07.81**

(51) Int. Cl.³: **B 01 F 5/04,** B 01 F 3/04,
C 02 F 3/20, C 12 M 1/04

(54) **Vorrichtung zum Begasen von Flüssigkeiten oder Suspensionen.**

(30) Priorität: **17.07.80 DE 3027035**

(43) Veröffentlichungstag der Anmeldung:
**27.01.82 Patentblatt 82/4**

(45) Bekanntmachung des Hinweises auf die Patenterteilung:
**19.09.84 Patentblatt 84/38**

(84) Benannte Vertragsstaaten:
**AT BE CH DE FR GB IT LI NL SE**

(56) Entgegenhaltungen:
**DE - A - 2 634 496**
**DE - A - 2 646 742**
**US - A - 2 616 676**

(73) Patentinhaber: **HOECHST AKTIENGESELLSCHAFT,**
**Postfach 80 03 20, D-6230 Frankfurt am Main 80 (DE)**

(72) Erfinder: **Müller, Gerhard, Feldbergstrasse 76,**
**D-6233 Kelkheim (Taunus) (DE)**
Erfinder: **Sell, Günther, Stettiner Strasse 84,**
**D-6234 Hattersheim am Main (DE)**

EP 0 044 498 B1

## Beschreibung

Gegenstand der Erfindung ist eine mischraumlose Vorrichtung zum Begasen von Flüssigkeiten oder Suspensionen bei chemischen oder biologischen Prozessen z. B. in der Fermentations- oder in der Abwassertechnik.

Neben einfachen Begasungssystemen wie z. B. Einstoffsysteme, sind auch Vorrichtungen z. B. Zweistoffsysteme wie Injektoren, Ejektoren, Strahlmischer, Strahldüsen usw. zum Begasen von Flüssigkeiten oder Suspensionen bekannt, bei denen die kinetische Energie eines Flüssigkeitsstrahles zum Zerteilen des Gases benutzt wird. Die Flüssigkeit wird bei diesem System (vergl. DE-A-2 634 496) über eine Treibstrahldüse und Gas über eine entsprechende Zuführung einem geschlossenen Mischraum zugeführt. Die Mischung findet also in einem »geschlossenen System« statt. Diese Systeme haben den Nachteil, daß ihre Effizienz bei Vergrößerung der Vorrichtung rasch abnimmt. Die Dispergierung des Gases erfolgt nämlich überwiegend in den Randzonen des Flüssigkeitsstrahles. Bei Vergrößerung des Volumenstromes wächst die Fläche der Randzone nicht entsprechend mit. Ein weiterer Nachteil der bekannten Begasungssysteme ist die unzulängliche Einmischung der Gasblasen bzw. des Flüssigkeit-Blasen-Gemisches in die umgebende Flüssigkeit, wodurch die Blasenkoaleszenz begünstigt wird.

Der Erfindung liegt demnach die Aufgabe zugrunde, eine Vorrichtung zu schaffen, die weitgehend unabhängig von ihrer Größe eine verbesserte Dispergierung und Verteilung der Gasblasen in Flüssigkeiten und Suspensionen ermöglicht.

Die Aufgabe wird durch eine Vorrichtung ohne Mischraum gelöst, die dadurch gekennzeichnet ist, daß eine erste Leiteinrichtung in geringem Abstand gegenüber der Öffnung eines Treibstrahlrohres angeordnet ist und sich über eine Gaszuführung auf einer zweiten Leitstrahleinrichtung abstützt.

Als erste Leiteinrichtung kann ein Kegel und als zweite Leiteinrichtung ein Kegelstumpf angeordnet sein, wobei der Abstand zwischen den Leiteinrichtungen 3 bis 50 mm und der Durchmesser des Kegels 1 bis 10% größer als der kleinere Durchmesser des Kegelstumpfes gewählt werden kann. Statt eines Kegels mit gerader Mantellinie ist auch ein Kegel mit konkaver Mantellinie möglich. Der Kegelwinkel des Kegelstumpfes kann 0 bis 20° größer als der Kegelwinkel des Kegels sein. Der Mantel des Kegelstumpfes kann asymptotisch in die Basisfläche des Kegelstumpfes einmünden, d. h. stetig in einen scheibenförmigen Ringbereich übergehen. Dabei kann dieser scheibenförmige Ringbereich ein Vielfaches der Grundfläche des Kegelstumpfes sein. Um den Kegelstumpf herum kann ein Leitorgan angeordnet sein, dessen Abstand zum Mantel des Kegelstumpfes mit zunehmendem Durchmesser abnimmt.

Der flüssige Treibstrahl wird durch die Leiteinrichtungen in einen schräg oder radial abströmenden Film aufgerissen, in dessen Grenzfläche sich infolge der Scherkräft die Gasblasen bilden. Gleichzeitig wird das Blasen-Flüssigkeits-Gemisch gleichmäßig in die zu begasende Flüssigkeit oder Suspension eingemischt.

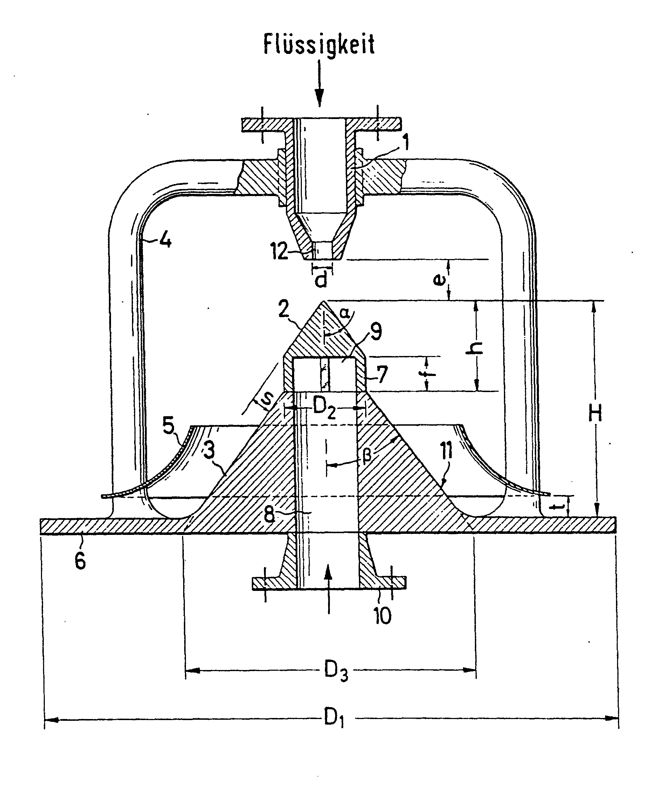

Im folgenden wird die Erfindung anhand der Figur, die einen schematischen Aufbau der Vorrichtung in beispielsweiser Ausführung zeigt, näher erläutert.

Die Vorrichtung zum Begasen besteht aus einem Treibstrahlrohr (1), den beiden Leiteinrichtungen (2 und 3) und der Gaszuführung (7). Das Treibstrahlrohr (1) ist so über der Leiteinrichtung (2), z. B. einem Kegel mit Winkel $\alpha = 5°$ bis 89° oder einer Platte, oder einer Kombination aus beiden angeordnet, daß der Treibstrahl durch die Leiteinrichtung (2) möglichst symmetrisch zu einem Flüssigkeitsfilm aufgerissen wird, der sich bis zur Abrißkante der Leiteinrichtung (2) stetig vergrößert. Der Abstand e zwischen Treibstrahlrohr (1) und Leiteinrichtung (2) kann Null bis 5 d, vorzugsweise 2 d sein, wobei der Durchmesser der Austrittsöffnung (12) wird nach Maßgabe der durchzusetzenden Flüssigkeitsmengen gewählt. Durchmesser zwischen 20 und 40 mm haben sich als brauchbar herausgestellt. Das Treibstrahlrohr (1) ist in bezug auf die Leiteinrichtung (2) mittels Bügel (4) fixiert, die auf der Leiteinrichtung (3) angeordnet sind. Die Leiteinrichtung (2) ist auf der Gaszuführung (7) angeordnet, die sich auf der Leiteinrichtung (3) abstützt. Die Leiteinrichtung (3) ist mit einer Bohrung (8) versehen, die mit der Gaszuführung (7), z. B. einem zylindrischen Ring mit Öffnungen (9) verbunden ist. (10) deutet einen Flansch zum Anschließen einer Gasleitung an. Die zweite Leiteinrichtung (3) ist so dimensioniert, daß sich zwischen ihrer Oberfläche und dem von der ersten Leiteinrichtung (2) kommenden Flüssigkeitsfilm ein Ringspalt mit der Dicke s ausbilden kann, in den das Gas über die Gaszuführung (7) eingezogen wird. Die Dicke des Ringspaltes wird durch die Gasmenge festgelegt. Dabei soll das Verhältnis Spaltdicke s zu dem Abstand f = 3 bis 50 mm der beiden Leiteinrichtungen (2 und 3) voneinander 0,1 bis 1 betragen. Die Gasgeschwindigkeit im Ringspalt sollte nicht größer als 50 m/sec betragen. In der Flüssigkeitsgrenzfläche und auf der Oberfläche der zweiten Leiteinrichtung (3) findet eine intensive Durchmischung von Treibstrahlflüssigkeit und Gas und damit die Blasenbildung statt. Auch an der zweiten Leiteinrichtung (3) vergrößert sich der Flüssigkeitsfilm stetig. Außerdem wird sekundär Flüssigkeit angesaugt und ebenfalls mit dem Gas vermischt. Bei Verwendung eines Kegelstumpfes als Leiteinrichtung (3) hat sich als vorteilhaft herausgestellt, den Mantel (11) asymptotisch in einen scheibenförmigen Ringbereich (6) einmünden zu lassen. Über diesen Ringbereich wird das Flüssigkeitsblasengemisch horizontal in die zu begasende Flüssigkeit oder Suspension eingeleitet, wobei eine weitgehend gleichmäßige Begasung ermöglicht wird. Es kann von Vorteil sein, um die Leiteinrichtung (3) herum ein Leitorgan (5) anzuordnen, wodurch in nicht koaleszierenden Systemen, z. B. Alkohol-Wasser-Gemischen, bei gleichem Energieaufwand Blasen mit kleinerem Durchmesser als in koaleszierenden Systemen (Wasser) erzeugt werden können. Es ist zweckmäßig, den Abstand des Leitorgans (5) zur Oberfläche der Leiteinrichtung (3) mit wachsendem Durchmesser zu verkleinern, wobei das Verhältnis des größten Durchmessers $D_3$ der Leiteinrichtung (3) zum kleinsten Abstand t des Leitorganes (5) zur Oberfläche der Leiteinrichtung (3) 3 bis 30 betragen soll. Das Verhältnis Durchmesser $D_1$ des scheibenförmigen Ringbereiches (6) zum Durchmesser d der Austrittsöffnung (12) des Treibstrahlrohres (1) kann 5 bis 100 und das des Durchmessers $D_2$ der Leiteinrichtung (2) zu d 2 bis 20 betragen. Das Verhältnis Höhe h, die sich aus der Höhe der Leiteinrichtung (2) und der Gaszuführung (7) ergibt zu Höhe H, die sich aus der Höhe der Leiteinrichtung (3) und der Höhe h ergibt, kann 0,01 bis 1 betragen.

## Patentansprüche

1. Mischraumlose Vorrichtung zum Begasen von Flüssigkeiten oder Suspensionen, dadurch gekennzeichnet, daß eine erste Leiteinrichtung (2) in geringem Abstand gegenüber der Austrittsöffnung (12) eines Treibstrahlrohres (1) angeordnet ist und sich über eine Gaszuführung (7) auf einer zweiten Leiteinrichtung (3) abstützt.

2. Vorrichtung nach Anspruch 1, dadurch gekennzeichnet, daß als erste Leiteinrichtung ein Kegel angeordnet ist, und als zweite Leiteinrichtung ein Kegelstumpf, wobei der Abstand zwischen den Leiteinrichtungen 3 bis 50 mm und der Durchmesser des Kegel 1 bis 10% größer als der kleinere Durchmesser des Kegelstumpfes ist.

3. Vorrichtung nach Anspruch 2, dadurch gekennzeichnet, daß der Kegelwinkel $\beta$ des Kegelstumpfes 0 bis 20° größer ist als der Kegelwinkel $\alpha$ des Kegels.

4. Vorrichtung nach Anspruch 2, dadurch gekennzeichnet, daß die Mantelfläche des Kegelstumpfes asymptotisch in einen scheibenförmigen Ringbereich (6) einmündet.

5. Vorrichtung nach Anspruch 2, dadurch gekennzeichnet, daß um den Kegelstumpf herum ein Leitorgan (5) angeordnet ist, wobei der Abstand zwischen Leitorgan und Mantelfläche des Kegelstumpfes mit zunehmendem Durchmesser abnimmt.

## Claims

1. A device for gassing liquids or suspensions, wherein a guide means (2) is located, at a short distance, opposite the outlet orifice (12) of a jet pipe (1) and is supported via a gas inlet (7) on a second guide means (3).

2. A device as claimed in claim 1, wherein the first guide means provided is a cone and the second guide means is a truncated cone, the distance between the guide means being 3 to 50 mm and the diameter of the cone being 1 to 10% greater than the smaller diameter of the truncated cone.

3. A device as claimed in claim 2, wherein the cone angle $\beta$ of the truncated cone is 0 to 20°C greater than the cone angle $\alpha$ of the cone.

4. A device as claimed in claim 2, wherein the shell surface of the truncated cone merges asymptotically into a disc-shaped annular zone (6).

5. A device as claimed in claim 2, wherein a guide element (5) is arranged around the truncated cone, the distance between the guide element and the shell surface of the truncated cone decreasing with increasing diameter.

## Revendications

1. Appareil sans volume mélangeur pour gazéifier des liquides ou des suspensions, caractérisé en ce qu'un premier dispositif déflecteur (2) est placé à une faible distance par rapport à l'orifice de sortie (12) d'un tube à jet de propulsion (1) et s'appuie par l'intermédiaire d'une arrivée de gaz (7) sur un second dispositif déflecteur (3).

2. Appareil selon la revendication 1, caractérisé en ce qu'il est prévu comme premier dispositif déflecteur un cône et comme second dispositif déflecteur un tronc de cône, l'espacement entre les dispositifs déflecteurs étant de 3 à 50 mm et le diamètre du cône étant supérieur de 1 à 10% au petit diamètre du tronc de cône.

3. Appareil selon la revendication 2, caractérisé en ce que l'angle de conicité $\beta$ du tronc de cône est supérieur de 0 à 20° à l'angle de conicité $\alpha$ du cône.

4. Appareil selon la revendication 2, caractérisé en ce que la surface périphérique du tronc de cône débouche asymptotiquement dans une zone annulaire en forme de disque (6).

5. Appareil selon la revendication 2, caractérisé en ce qu'il est prévu autour du tronc de cône un organe déflecteur (5), l'espacement entre l'organe déflecteur et la surface périphérique du tronc de cône diminuant quand le diamètre augmente.

**Flüssigkeit**